# EUROPÄISCHE PATENTSCHRIFT

(11) **EP 3 600 481 B1**
(45) Veröffentlichungstag und Bekanntmachung des Hinweises auf die Patenterteilung: **17.11.2021**
(21) Anmeldenummer: 18713838.3
(22) Anmeldetag: 20.03.2018
(51) Int. Cl.: A61M 1/14, A61M 1/36, H01L 41/113

(54) **MEDIZINISCHE EINRICHTUNG MIT ADDITIV AUFGEBRACHTEM WANDLER**
MEDICAL APPARATUS COMPRISING A CONVERTER APPLIED BY ADDITIVE TECHNIQUES
ÉQUIPEMENT MÉDICAL DOTÉ D'UN TRANSDUCTEUR APPLIQUÉ PAR FABRICATION ADDITIVE

(30) Priorität: 24.03.2017 DE 102017106402
(43) Veröffentlichungstag der Anmeldung: 05.02.2020
(73) Patentinhaber: Fresenius Medical Care Deutschland GmbH, 61352 Bad Homburg v.d.H. (DE)
(72) Erfinder: HEIDE, Alexander, 65817 Eppstein (DE); NIKOLIC, Dejan, 65812 Bad Soden (DE)
(74) Vertreter: Bobbert & Partner Patentanwälte PartmbB
(86) Internationale Anmeldenummer: PCT/EP2018/057037
(87) Internationale Veröffentlichungsnummer: WO 2018/172364

(56) Entgegenhaltungen:
- US-A1- 2009 012 452
- US-A1- 2011 214 504

## Beschreibung

Die vorliegende Erfindung betrifft eine medizinische Einrichtung gemäß dem Oberbegriff des Anspruchs 1. Sie betrifft ferner ein Herstellverfahren für eine medizinische Einrichtung gemäß dem Oberbegriff des Anspruchs 12 sowie eine Behandlungsvorrichtung gemäß dem Oberbegriff des Anspruchs 14.

Bekannte Blutbehandlungsvorrichtungen werden zur Blutbehandlung mit wenigstens einer medizinischen Einrichtung verbunden, beispielsweise in Gestalt einer Blutkassette, in welcher Blut behandelt oder vorübergehend gespeichert wird. Als Blutkassetten ausgestaltete medizinische Einrichtungen sind aus der DE 10 2009 018 664 A1 bekannt.

In der US 2011/0214504 A1 sind Einwegdrucksensorsysteme und Packarten hierzu offenbart.

Aus der US 2009/012452 A1 sind Dialysierflüssigkeitsmesssysteme mit leitenden Sensoren bekannt.

Aufgabe der vorliegenden Erfindung ist, eine weitere medizinische Einrichtung anzugeben. Ferner sollen ein Herstellverfahren für eine solche Einrichtung und eine Behandlungsvorrichtung zu deren Verwendung vorgeschlagen werden.

Diese Aufgabe kann durch eine medizinische Einrichtung mit den Merkmalen des Anspruchs 1, ein Verfahren mit den Merkmalen des Anspruchs 12 und eine Behandlungsvorrichtung mit den Merkmalen des Anspruchs 14 gelöst werden.

Erfindungsgemäß wird somit eine medizinische Einrichtung mit wenigstens einem Hartteil mit vollständigen oder unvollständigen Fluidbahnen zum Führen eines medizinischen Fluids, insbesondere Blut, im Hartteil oder durch das Hartteil hindurch, angegeben.

Die medizinische Einrichtung weist ferner wenigstens einen Wandler auf. Der Wandler ist angeordnet zum Messen einer Eigenschaft des medizinischen Fluids oder eines anderen Fluids, während dieses in einer der Fluidbahnen vorliegt. Alternativ oder ergänzend ist der Wandler angeordnet zum Messen einer Eigenschaft der medizinischen Einrichtung oder einer Einwirkung wie einen von außen oder innen aufgebrachten Druck auf letztere.

Der Wandler ist zumindest in einem Abschnitt hiervon mittels genau eines oder wenigstens eines additiven Aufbringungsverfahrens, vorzugsweise eines Druckverfahrens - direkt oder indirekt - auf das Hartteil aufgebracht und/oder erzeugt.

Das erfindungsgemäße Verfahren dient dem Herstellen einer erfindungsgemäßen medizinischen Einrichtung. Es umfasst das Bereitstellen eines Hartteils der medizinischen Einrichtung, welches Fluidbahnen, ein Fluidsystem, oder Abschnitte hierfür, für ein medizinisches Fluid aufweist.

Das Verfahren umfasst ferner das Aufbringen wenigstens eines Wandlers (zumindest in einem Abschnitt hiervon) auf das Hartteil mittels wenigstens eines additiven Aufbringungsverfahrens, vorzugsweise eines Druckverfahrens.

Die erfindungsgemäße Behandlungsvorrichtung ist verbunden mit dem Multipolstecker (dieser Begriff kann im Umfang der vorliegenden Erfindung auch durch "Multistecker" ausgetauscht werden) einer erfindungsgemäßen medizinischen Einrichtung.

Die erfindungsgemäße Behandlungsvorrichtung kann konfiguriert sein zum Empfangen und Verarbeiten der mittels des Multipolverbinders empfangenen Signale.

Die erfindungsgemäße Behandlungsvorrichtung kann mit einer erfindungsgemäßen medizinischen Einrichtung verbunden sein.

Bei allen Ausführungen hierin ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale aufweisen. Dabei können die hierin genannten Merkmale in beliebiger Kombinationen Gegenstand von erfindungsgemäßen Ausführungsformen sein, sofern der Fachmann eine konkrete Kombination nicht als technisch unmöglich erkennt. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

In einigen beispielhaften, erfindungsgemäßen Ausführungsformen sind unter dem Hartteil (hierin auch als Hartkörper oder als Substrat bezeichnet) der in der Regel in einem Spritzgussverfahren hergestellte und daher hierin als "hart" bezeichnete Korpus der medizinischen Einrichtung, welcher mit einer vergleichsweise "weichen" Folie bedeckt sein kann, und/oder seine Anhänge wie Schläuche usw., zu verstehen. Das Hartteil kann aus PP (Polypropylen), PE (Polyethylen), PA, ABS, PMMA, PC, PVC oder anderen dem Fachmann hinlänglich bekannten Polymeren oder anderen Materialien gefertigt sein. Es kann aus Isolatormaterialien, wie insbesondere z. B. Keramik, gefertigt sein.

In einigen beispielhaften, erfindungsgemäßen Ausführungsformen sind unter dem Hartteil (hierin auch als Hartkörper oder als Substrat bezeichnet) nur der in der Regel in einem Spritzgussverfahren hergestellte und daher hierin als "hart" bezeichnete Korpus der medizinischen Einrichtung, welcher mit einer vergleichsweise "weichen" Folie bedeckt sein kann, zu verstehen, nicht aber auch seine Anhänge wie Schläuche usw.

Im Rahmen der vorliegenden Beschreibung werden die Begriffe "Sensor" und "Sensoranordnung" gleichbedeutend für ein System mit oder aus einem Wandler, Signalübertragungskomponenten und Auswerteeinheit verwendet. Ein Wandler ist dabei vorzugsweise als ein einrichtungsseitig vorgesehener Abschnitt des Sensors zu verstehen, der mit dem Messmedium - vorzugsweise direkt oder indirekt - in Kontakt und/oder in Messbeziehung steht. Er wandelt das Ergebnis seiner Messung in geeignete Signale, z. B. Strom- oder Spannungssignale, um. Die ebenfalls zur Sensoranordnung zählenden Signalübertragungskomponenten leiten das Ergebnis seiner Messung an die Auswerteeinheit weiter, die im Stand der Technik üblicherweise maschinenseitig vorgesehen ist.

In einigen beispielhaften, erfindungsgemäßen Ausführungsformen umfasst das Aufbringungsverfahren das Aufbringen von leitfähiger Tinte oder besteht hieraus.

Unter leitfähiger Tinte sind hier Fluide mit beispielsweise Nano- oder Mikropartikeln zu verstehen, die, wenn sie geeignet dicht zusammen aufgebracht werden, eine funktionale Bahn ergeben, wie z. B. eine Leiterbahn (hierin auch als Elektrode bezeichnet), über die unter anderem Signale übertragen werden können. Im Sinne der Erfindung kommen aber auch andere Materialien als leitfähige Tinte in Frage. Leitfähige Tinte kann idealerweise biokompatibel sein, muss aber nicht. Es können ergänzend oder alternativ lebende Zellen, Proteine etc. Bestandteil der- ggf. auch - durch sie leitfähig gemachten Tinte sein.

Die leitfähige Tine oder das alternative Material kann in beliebigen Aggregatszuständen verwendet werden. Unter einem Aggregatszustand im Sinne der vorliegenden Erfindung kann in einigen Ausführungsformen ein festes oder flüssiges Hydrogel verstanden werden, in welches ein Biomarker eingelagert ist. Es können auch gefrorene (also feste) Substanzen oder mikroinkapsulierte Wirkstoffe/Reagenzien verarbeitet werden. Auch können Bahnen per Sublimation/Kondensation aus der Gasphase abgeschieden werden.

Unter leitfähiger Tinte sind hier beispielsweise auch Liquide zu verstehen, die Karbon, leitende Polymere, Metallpartikel und/oder Kombinationen hiervon aufweisen, ferner metallisierte Tinte.

Die aus dem Stand der Technik bekannte Technik des Aerosoljet-Druckens (siehe hierzu die EP 2 559 656 A1) kommt hier als Beispiel für ein additives Aufbringen und insbesondere zum Bedrucken in Betracht. Die dort offenbarte Technik und die darin beschriebenen Vorrichtungen zum Ausführen dieser Technik eignen sich hierfür, insbesondere auch aufgrund der geometrischen Ausführung der Druckdüse (Nozzle tip), welche die notwendige Anzahl an Freiheitsgraden in der Bewegung beim Druckvorgang erlaubt.

Die vorliegende Erfindung ist natürlich nicht auf die Anwendung des Aerosoljets begrenzt. Der Fachmann erkennt, dass alle additiven und/oder maskierungsfreien Druckverfahren, insbesondere mittels welcher leitfähige Tinte im Sinne dieser Beschreibung appliziert werden kann, von der vorliegenden Erfindung umfasst sind.

In einigen beispielhaften, erfindungsgemäßen Ausführungsformen umfasst ein Aufbringungsverfahren ein Aufbringen in mehreren Schichten.

In einigen beispielhaften, erfindungsgemäßen Ausführungsformen umfasst das Aufbringungsverfahren eine Sequenz oder Aufeinanderfolge von eigenständigen Aufbringungsverfahren oder von Schritten, die zu ein und demselben Aufbringungsverfahren zählen.

In einigen beispielhaften, erfindungsgemäßen Ausführungsformen umfasst das additive Aufbringen sog. "additive manufacturing" bzw. generative Fertigungsverfahren und/oder besteht hieraus.

Insbesondere können hierunter die folgenden Verfahren verstanden werden:
- selektives Laserschmelzen (SLM),
- selektives Lasersintern (SLS),
- Selective Heat Sintering (SHS),
- Binder Jetting (Verfestigen von Pulvermaterial mittels Binder)
- Elektronenstrahlschmelzen (Electron Beam Melting = EBM)
- Fused Deposition Modelling (FDM oder auch Fused Filament Fabrication (FFF)),
- Auftragschweißen bzw. Cladding,
- Wax Deposition Modelling (WDM),
- Contour Crafting,
- Metall-Pulver-Auftragsverfahren (MPA),
- Kaltgasspritzen
- Stereolithografie (SLA) + Mikro-SLA,
- Verfahren, welche Digital Light Processing (DLP) zur Belichtung nutzen und
- Liquid Composite Moulding (LCM),
- Laminated Object Modelling (LOM),
- 3D-Siebdruck von Metallen und
- Lichtgesteuerte Elektrophoretische Abscheidung.

In einigen beispielhaften, erfindungsgemäßen Ausführungsformen umfasst das Aufbringungsverfahren ein maskierungsfreies Aufbringen oder besteht hieraus.

In einigen beispielhaften, erfindungsgemäßen Ausführungsformen ist unter einem maskierungsfreien Aufbringen ein Aufbringen ohne Maske oder Schablone zu verstehen.

In einigen beispielhaften, erfindungsgemäßen Ausführungsformen ist das Aufbringungsverfahren kein Maskentintendruck, kein "ink stencil printing", kein "screen printing" und/oder kein Fotolithographie-Verfahren, insbesondere jeweils nicht in einem kontinuierlichen Verfahren.

In einigen beispielhaften, erfindungsgemäßen Ausführungsformen ist oder umfasst das Aufbringungsverfahren kein Kleben oder Klebeverfahren, kein Schweißen und/oder kein Löten.

In einigen beispielhaften, erfindungsgemäßen Ausführungsformen ist unter einem maskierungsfreien Aufbringen ein Verzicht auf jede - anschließend zu entfernende - Hilfsschicht oder Blende zu verstehen.

Unter "Maskierung" kann hierin ein Abschatten oder Schützen in geeigneter Form von Bereichen, welche nicht beschichtet werden dürfen, verstanden werden. Diese Maskierung geschieht z. B. durch Blenden (z. B. Siebdruck, Sprühlackierung etc.) oder durch "Lack"-Masken (z. B. Wafer, Platinen-Fertigung etc.).

In einigen beispielhaften, erfindungsgemäßen Ausführungsformen weist die medizinische Einrichtung eine Vielzahl von Wandlern, d. h. zwei oder mehr, auf, welche, jeweils zumindest in einem Abschnitt hiervon, mittels wenigstens eines additiven Aufbringungsverfahrens, vorzugsweise eines Druckverfahrens - direkt oder indirekt - auf das Hartteil aufgebracht sind.

In einigen beispielhaften, erfindungsgemäßen Ausführungsformen wurden additiv weitere Abschnitte einer Sensoranordnung oder einer Vielzahl von Sensoranordnungen aufgebracht, vorzugsweise unter Einsatz desselben Aufbringungsverfahrens.

In einigen beispielhaften, erfindungsgemäßen Ausführungsformen wurden die mittels eines additiven Aufbringungsverfahrens aufgebrachten Abschnitte der einen oder der Vielzahl der Sensoranordnungen im selben Produktionsschritt aufgebracht.

In einigen, beispielhaften, erfindungsgemäßen Ausführungsformen umfassen die mittels eines additiven Aufbringungsverfahrens aufgebrachten Abschnitte zusätzlich zum wenigstens einen Wandler wenigstens Leiterbahnen, Elektroden, einen Multipolstecker oder jeweils eine Vielzahl hiervon.

Erfindungsgemäß sind der oder die Wandler zum Messen oder Bestimmen von Druck ausgestaltet oder konfiguriert.

In einigen beispielhaften, erfindungsgemäßen Ausführungsformen bewirkt das Aufbringungsverfahren eine 2-dimensionale oder eine 3-dimensionale Aufbringung, und/oder die mittels eines additiven Aufbringungsverfahrens aufgebrachten Wandler und/oder weiteren Abschnitte wurden mittels des Aufbringungsverfahrens 2-dimensional oder 3-dimensional aufgebracht.

In einigen beispielhaften, erfindungsgemäßen Ausführungsformen wurde wenigstens ein Multipolstecker ebenfalls mit dem erstem Aufbringungsverfahren oder zeitgleich zu diesem aufgebracht.

In einigen beispielhaften, erfindungsgemäßen Ausführungsformen ist zum Aufbringen der mittels additiver Aufbringungsverfahren aufgebrachten Abschnitte ein zweites Aufbringungsverfahren, optional ähnlich dem ersten, zum Einsatz gekommen.

In einigen beispielhaften, erfindungsgemäßen Ausführungsformen wurden Abschnitte des oder der Wandler oder der weiteren Abschnitte der wenigstens einen Sensoranordnung mittels Spritzgussverfahrens aufgebracht, z. B. als Zwei-Komponentendruck, z. B. mittels leitfähiger Polymere.

In einigen beispielhaften, erfindungsgemäßen Ausführungsformen ist oder umfasst der Wandler keine LED (light emitting diode) oder OLED (organic light emitting diode) und/oder keinen Photodetektor.

In einigen beispielhaften, erfindungsgemäßen Ausführungsformen ist die medizinische Einrichtung eine Blutkassette. Sie kann zum einmaligen Einsatz bei der Blutbehandlung vorgesehen sein.

In einigen beispielhaften, erfindungsgemäßen Ausführungsformen umfasst das Verfahren weiter wenigstens einen der folgenden Schritte: Nachbearbeitungsschritte, insbesondere an den Leiterbahnen oder Elektroden, wie z. B. Schleifen, Polieren, Isolieren, Aufbringen weiterer funktionaler Schichten aus anderem Material; additives Aufbringen, z. B. durch Bedrucken, der Signalanbindung an die Maschinenschnittstelle; additives Aufbringen, z. B. durch Bedrucken, des Multipolsteckers.

Sowohl die Leiterbahn (hierin auch als Signalleiter bezeichnet) als auch der Multipolstecker können planar (also 2-dimensional) oder 3-dimensional mit einer oder mehreren additiven und maskierungsfreien Drucktechniken aufgebracht werden. Auch hier sind ein oder mehrere Nachbearbeitungsschritte wie vorstehend bei den Leiterbahnen beschrieben, optional umfasst.

In einigen beispielhaften, erfindungsgemäßen Ausführungsformen umfasst das Verfahren weiter das Zusammenfügen von zwei oder mehr, wie hierin beschrieben, mittels additiven Aufbringens bearbeiteter Abschnitte oder Komponenten der medizinischen Einrichtung.

In einigen erfindungsgemäßen, beispielhaften Ausführungsformen ist die erfindungsgemäße medizinische Einrichtung ein Schlauch, ein Schlauchsystem, ein Schlauchset, eine Blutkassette oder jeweils ein Teil hiervon.

Ein "medizinisches Fluid" im Sinne der vorliegenden Erfindung schließt jede medizinische Flüssigkeit und/oder jedes medizinische Gas sowie beliebige Kombinationen ein. Vorzugsweise handelt es sich bei dem Fluid um Blut.

Eine erfindungsgemäße medizinische Einrichtung kann eine Einmalkomponente oder ein Einmalartikel ("Disposable") sein, welcher beispielsweise aus einem Kunststoffmaterial gefertigt ist.

Die erfindungsgemäße medizinische Einrichtung kann mittels eines Spritzgussverfahrens hergestellt sein.

Die erfindungsgemäße medizinische Einrichtung kann über Flüssigkeits- und/oder Gasanschlüsse, über halboffene Kanäle und/oder Kammern verfügen. Ein oder mehrere Abdeckungselemente, wie beispielsweise Membranen oder Folien, können für den Abschluss und/oder die Abdichtung der Kanäle und Kammern sorgen.

Die Blutbehandlung, für welche die medizinische Einrichtung verwendet wird, kann beispielsweise ein Dialysierverfahren, eine Hämodialyse, Hämofiltration, Hämodiafiltration und dergleichen sein.

In bestimmten beispielhaften, erfindungsgemäßen Ausführungsformen ist die medizinische Einrichtung eine Blutkassette. Das Hartteil ist in diesen Fällen ein Kassettenkörper, Kassettengrundkörper oder ein Schlauchabschnitt, alternativ jedoch kein Schlauchabschnitt.

In bestimmten beispielhaften, erfindungsgemäßen Ausführungsformen weist die medizinische Einrichtung ferner wenigstens zwei Konnektoren für Pumpschlauchsegmente für peristaltische Pumpen, mit oder ohne den Pumpschlauchsegmenten, auf.

In bestimmten beispielhaften, erfindungsgemäßen Ausführungsformen weist die medizinische Einrichtung eine Single-Needle-Sterilmembran auf.

In bestimmten beispielhaften, erfindungsgemäßen Ausführungsformen weist die Vorrichtung Dehnungsmessstreifen (DMS Sensoren) auf. Die Verformung des Untergrundes führt zur Längenänderung der Messbahn und ändert deren Widerstand. Alternativ könnte auch ein Material mit piezoelektrischen Eigenschaften aufgedruckt werden, z. B. PVDF (Polymer) oder Piezokeramik. Mit Hilfe des Piezoeffekts würden bevorzugt (schnelle) Druckänderungen oder Schwingungen gemessen.

In bestimmten beispielhaften, erfindungsgemäßen Ausführungsformen ist die Behandlungsvorrichtung ausgestaltet als Blutbehandlungsvorrichtung, insbesondere als Apheresevorrichtung oder Dialysevorrichtung, insbesondere als Hämofiltrationsvorrichtung, als Hämodiafiltrationsvorrichtung, als Filtrationsvorrichtung oder als Vorrichtung zum extrakorporalen Gasaustausch.

In bestimmten beispielhaften, erfindungsgemäßen Ausführungsformen weist die Behandlungsvorrichtung Aktuatoren wie z. B. Pumpen oder Ventile und/oder mechanische oder nicht-mechanische Schnittstellen zum Einwirken mittels dieser Aktuatoren auf die medizinische Einrichtung auf.

In bestimmten beispielhaften, erfindungsgemäßen Ausführungsformen weist die Behandlungsvorrichtung nicht den Wandler auf, der erfindungsgemäß auf der medizinischen Einrichtung angeordnet ist. Weist die medizinische Einrichtung beispielsweise einen Drucksensor als Wandler auf, so ist in die Signalkommunikation zwischen Wandler und Ausgabe eines mittels diesem gemessenen Signals kein maschinenseitiger Drucksensor eingebunden, insbesondere kein vollständiger Drucksensor, insbesondere kein Drucksensor, der mechanisch auf die medizinische Einrichtung einwirken würde.

In bestimmten beispielhaften, erfindungsgemäßen Ausführungsformen umfasst der Multipolverbinder der Behandlungsvorrichtung eine Spannungsleitung, mittels welcher die medizinische Einrichtung mit elektrischer Spannung versorgt wird. Die elektrische Versorgung kann der Datenauslese oder dem Auslesen von Daten ebenso wie dem Betrieb der Sensoren der medizinischen Einrichtung dienen.

Beispiele für Wandler im Sinne der vorliegenden Erfindung umfassen insbesondere:
1) Wandler für kapazitive Messungen: Zwischen zwei leitenden Oberflächen wird ein Dielektrikum angebracht. Die zu messende Eigenschaft hat eine Wechselwirkung mit der Dielektrizitätskonstante des Dielektrikums und kann über die Kondensatoreigenschaften bestimmt werden. Mit dieser Art des Wandlers können u. a. bestimmt werden: Leitfähigkeit, Level (Füllstand bzw. Vorhandensein einer Flüssigkeit), Druck, Abstand (Näherungssensor).
2) Wandler für Widerstandsmessung: Eine äußere Wirkung auf einen Draht beeinflusst dessen Widerstand. Mit dieser Art des Wandlers können u. a. bestimmt werden: Temperatur, Druck (Dehnungsmessstreifen DMS), Gewicht, Kraft, Weg, Kontakt (ja, nein).
3) Wandler mittels Piezoeffekt: Ein Piezodot (z. B. PVDF) wird auf die Oberfläche einer Messzelle gedruckt. Messprinzip mittels Druckaufnahme, d.h. direktes Wandeln von äußerem Druck in Spannung, oder mittels Ultraschallerzeugung (räumliche Vermessung (Laufzeit) bzw. Dichtemessung (Amplitude) in Transmission mit einem zweiten Piezodot als Empfänger oder in Reflexion mit nur einem Piezodot. Mit dieser Art des Wandlers können u. a. bestimmt werden: Lufterkennung, Flussmessung, Bluterkennung, räumliche Vermessung (vgl. Unterwassermikrophone, Schrauben mit aufgebrachter Piezo-Drucksensorik zur Spannkraftüberwachung).
4) Magneto-induktive Wandler: Bekannter Disposable MID-Sensor zur Messung von Fluss oder Leitfähigkeit - in dieser Variante sind die Elektroden im Disposable nicht eingelegt und umspritzt, sondern aufgedruckt. Das Prinzip des Sensors ist z. B. in der WO2011/113838 beschrieben.
5) Optische Wandler: Ein Reagenz wird auf die Messoberfläche aufgedruckt. Sie wird entweder von außen oder von einem ebenfalls aufgedruckten optischen Emitter (Diode) bestrahlt. Die Eigenschaften der Reagenz bezüglich der Reflexion, Absorption, Lumineszenz und/oder Fluoreszenz sind von der zu vermessenden Größe abhängig. Über einen Fotodetektor, welcher die entsprechende Intensität aufnimmt, kann die Information gewonnen werden. Dabei kann der Fotodetektor vorzugsweise maschinenseitig angebracht sein, das gewandelte Signal wird über Lichtleiter zum Multipolstecker und von dort auf die Maschinenseite geleitet. Mit dieser Art des Wandlers können u. a. bestimmt werden: Verfärbung durch Temperatur, Druck, chemische Veränderung, pH, pO2, Glukosekonzentration (Hydrogel Optrode).

Einige oder alle erfindungsgemäßen Ausführungsformen können einen oder mehrere der oben oder im Folgenden genannten Vorteile aufweisen.

Moderne medizinische Systeme zur Blutbehandlung weisen üblicherweise eine Blutbehandlungsvorrichtung (auf der sogenannten "Maschinenseite") und daran anbringbare medizinische Einrichtungen (auf der sogenannten "Einrichtungsseite") auf. Ist die medizinische Einrichtung ein Einwegartikel oder Disposable, so ist hierin auch von der sogenannten "Disposableseite" die Rede. Obgleich die vorliegende Erfindung nicht auf Disposables beschränkt ist, wird hierin exemplarisch für medizinische Einrichtungen auch auf Disposables Bezug genommen, ohne dass dies beschränkend zu verstehen ist. Die zu einer Blutbehandlungssitzung verwendeten Einwegartikel werden aus Gründen der Hygiene nach der Blutbehandlungssitzung entsorgt, da sie im Kontakt mit Blut des Patienten standen oder gestanden haben können.

Zur Überwachung der Behandlung sind Sensoren und Aktoren erforderlich, die auf dem Einwegartikel bzw. der medizinischen Einrichtung Parameter messen bzw. auf die Einrichtung einwirken.

Eine Funktionalisierung der Einwegartikel selbst, also die Integration der gesamten zum Überwachen eines konkreten Parameters jeweils erforderlichen Sensorik in den Einwegartikeln, hat sich bisher zumeist als wirtschaftlich unrentabel herausgestellt, da die vergleichsweise teuren Sensorbauteile nach jeder Verwendung verworfen werden würden. Deshalb sind herkömmliche Einwegartikel zur medizinischen Blutbehandlung zumeist sehr einfach gestaltet, bestehen im Wesentlichen aus dem Schlauch zur Durchleitung des Bluts. Sensoren tragen sie aus dem vorstehend genannten Grund i. a. R. nicht.

Diese Sensoren und Aktoren sind im Stand der Technik folglich weitgehend maschinenseitig vorgesehen. Von dort wirken sie über eine Vielzahl von Sensor- und/oder Aktuatorschnittstellen auf die medizinische Einrichtung ein oder stehen mit dieser in Wechselwirkung. Diese Schnittstellen bestimmen in der Regel den Formfaktor und führen dazu, dass medizinische Systeme des Standes der Technik nicht beliebig klein ausgeführt werden können.

Die vorliegende Erfindung erlaubt es nun hingegen erstmals vorteilhaft, eine kostengünstige und damit wirtschaftliche Alternative zur Funktionalisierung von medizinischen Einrichtungen und auch Einwegartikeln bereitzustellen. Sie erlaubt damit vorteilhaft die Miniaturisierung der eingesetzten Blutbehandlungssysteme. Deren Miniaturisierung ist nicht zuletzt deshalb wünschenswert, da dies zur Reduzierung sowohl der extrakorporalen Blutmenge als auch des Aufwands für die extrakorporale Förderung und Hydraulik beiträgt.

Im Gegensatz zum Stand der Technik, in dem die Funktionalisierung durch die Integration der individuellen Sensorkomponenten in die Einwegartikel mit den vorstehend genannten Nachteilen versucht wurde, stellt die Erfindung, wie vorstehend beschrieben, eine Lösung zur wenig aufwändigen Verlagerung von Sensorkomponenten auf die medizinische Einrichtung dar.

Vorteilhaft ist ferner, dass mit der vorliegenden Erfindung eine Methode zur Verfügung gestellt wird, bei der alle einrichtungsseitig angeordneten Sensorkomponenten mit demselben Produktionsverfahren integriert werden können, was den Fertigungsaufwand weiter senken kann.

Die vorliegende Erfindung erlaubt dabei wahlweise eine vollständige Funktionalisierung oder eine nur teilweise Funktionalisierung der erfindungsgemäßen Einrichtung, die aufgrund der besonderen Art, wie die Wandler auf die Einrichtung verlagert werden, ohne nennenswerten wirtschaftlichen Verlust gemeinsam mit dem Wandler verworfen werden kann.

Unter einer teilweisen Funktionalisierung wird hierin beispielsweise im Falle eines Wandlers verstanden, dass die Kernaufgabe des Wandlers, also das Erfassen einer physikalischen, chemischen oder anderen Größe sowie das Wandeln dieser Größe in eine elektro-magnetisch übertragbare Substitutgröße, auf der medizinischen Einrichtung selbst, entweder invasiv (d. h. im Kontakt mit Blut oder einer Behandlungsflüssigkeit) oder nicht invasiv geschieht, jedoch z. B. die Verarbeitung des Signals sowie die Interpretation und weitere Schritte, die höherwertige, z. B. integrierte elektronische, Bauteile erfordert, auf Maschinenseite erfolgen. Ein Teil oder die gesamte Verarbeitung des Signals kann in einigen Fällen jedoch auch komplett auf der Einrichtungsseite erfolgen. Bei letzterer Ausführungsform der vorliegenden Erfindung wird von einer vollständigen Funktionalisierung gesprochen: Wenigstens eine Sensoranordnung oder ein Sensor liegt vollständig additiv auf die Einrichtung aufgebracht auf letzterer vor. Dabei können die einrichtungsseitigen Komponenten einer Sensoranordnung oder eines Sensors erfindungsgemäß alle durch dasselbe maskenfreie und additive Produktionsverfahren aufgebracht werden, wie vorstehend ausgeführt.

Von Vorteil ist ferner, dass, wie erfindungsgemäß auch vorgeschlagen, Einwegartikel, wenn sie mit individuellen vollständigen Sensoren ausgestattet werden, somit vollständig funktionalisiert werden können. Das hierin vorgestellte Verfahren zum Aufbringen von Wandlern oder anderen Komponenten von Sensoren macht dies möglich.

Die teilweise Funktionalisierung, und damit verbunden der Verbleib der komplexen, zur Nachverarbeitung und Auswertung der Signale notwendigen Elektronik auf der Maschinenseite, ist dadurch vorteilhaft, dass nur die zumeist einfacher gestalteten Komponenten der jeweiligen Sensoren auf der Einrichtung angeordnet sind, die durch die gemeinsame additive und maskierungsfreie Drucktechnik angebracht werden.

Die Funktionalisierung ist ferner wirtschaftlich von Vorteil, da die Herstellungskosten durch die gemeinsame Verwendung derselben Produktionstechnologie, idealerweise im selben Produktionsschritt für alle einrichtungsseitig angeordneten Sensorkomponenten, äußerst kostengünstig erfolgen kann.

Da das erfindungsgemäße Produktionsverfahren vorzugsweise ein additives, maskierungsfreies Druckverfahren ist, kann eine veränderte Version der Einrichtung, beispielsweise mit neuen Sensorgeometrien, optional sogar allein durch neues Aufspielen des entsprechenden Datensatzes in der Software produziert werden. Es ist keine Veränderung an der Produktionshardware notwendig, wie beispielsweise die Neuanschaffung eines Spritzgusswerkzeugs, das in der Produktion der medizinischen Einrichtung mittels Spritzguss erforderlich wäre. Auch dieser Aspekt führt zu einer vereinfachten Fertigung und zu verbesserter Wirtschaftlichkeit.

Ein weiterer Vorteil der Erfindung zu herkömmlichen (nicht-funktionalisierten) Systemen besteht darin, dass die zu messende physikalische oder chemische Größe nicht mechanisch auf die Maschinenseite geführt werden muss, wie das beispielsweise bei bekannten Drucksensoren der Fall ist, bei denen die zu messenden Druckverhältnisse über eine pneumatische Leitung zu einer Messmembran geführt werden, welche nicht disposable ist und maschinenseitig angeordnet ist. Um diese vor Flüssigkeit zu schützen, sind regelmäßig aufwändige Schutzmembranen notwendig, welche als Transducerprotector (TP) bezeichnet werden. Erfindungsgemäß können also alle mechanischen Schnittstellen zur Maschine entfallen. Alle zu messenden Größen können mittels der integrierten Teile der Sensorik zumindest in analoge elektromagnetische Signale (Strom, Spannung, optische Signale) gewandelt und erst in dieser Form zur Verarbeitungs- und Auswerteeinheit auf der Maschinenseite übermittelt werden. Zur Ankopplung der gesamten einrichtungsseitigen Sensorik ist nur noch eine entsprechende mehrkanalige elektromagnetische und/oder optische Schnittstelle notwendig (Multipolstecker), was eine signifikante Verkleinerung derselben erlaubt.

Ein weiterer Vorteil besteht im Multistecker oder Multipolstecker der Behandlungsvorrichtung. Auch dieser kann dazu beitragen, dass keine vollständigen Sensoren mehr in der Behandlungsvorrichtung existieren müssen, die dann mechanisch zur Messung der jeweiligen Größe auf die medizinische Einrichtung, etwa eine Disposablekassette, einwirken. Dadurch kann das Baumaß stark reduziert werden und die Maschinenseite wird zu einer "Docking Station", d.h. erst durch die Wahl des Typs der medizinischen Einrichtung und evtl. die Anwahl des entsprechenden Steuerprogramms in der Kontrolleinrichtung der Behandlungsvorrichtung wird die Therapie und das damit verbundenen Therapieverfahren festgelegt.

Ferner werden Quellen für Maschinen-Patienten Kontaminierung beseitigt: Druckmessung in der Maschine - die im Stand der Technik z. B. durch Sterilmembrane abgesichert werden müssen.

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigen:
- Fig. 1: ein medizinisches System zur Blutbehandlung mit einer medizinischen Einrichtung gemäß der vorliegenden Erfindung als teilfunktionalisiertes Disposable;
- Fig. 2: eine medizinische Einrichtung gemäß einer zweiten beispielhaften Ausführungsform der vorliegenden Erfindung;
- Fig. 3: einen zu Fig. 2 diskutierten Druckwandler in einer vergrößerten Perspektive;
- Fig. 4: einen weiteren zu Fig. 2 diskutierten Druckwandler in einer vergrößerten Perspektive;
- Fig. 5: ein Ausgangsprodukt des erfindungsgemäßen Produktionsprozesses zum Fertigen einer medizinischen Einrichtung gemäß der vorliegenden Erfindung;
- Fig. 6: das Ausgangsprodukt der Fig. 5 nach erfindungsgemäßem Aufbringen von Wandlern; und
- Fig. 7a, 7b: einen wie in Fig. 2 bereits gezeigten Multistecker oder Multipolstecker.

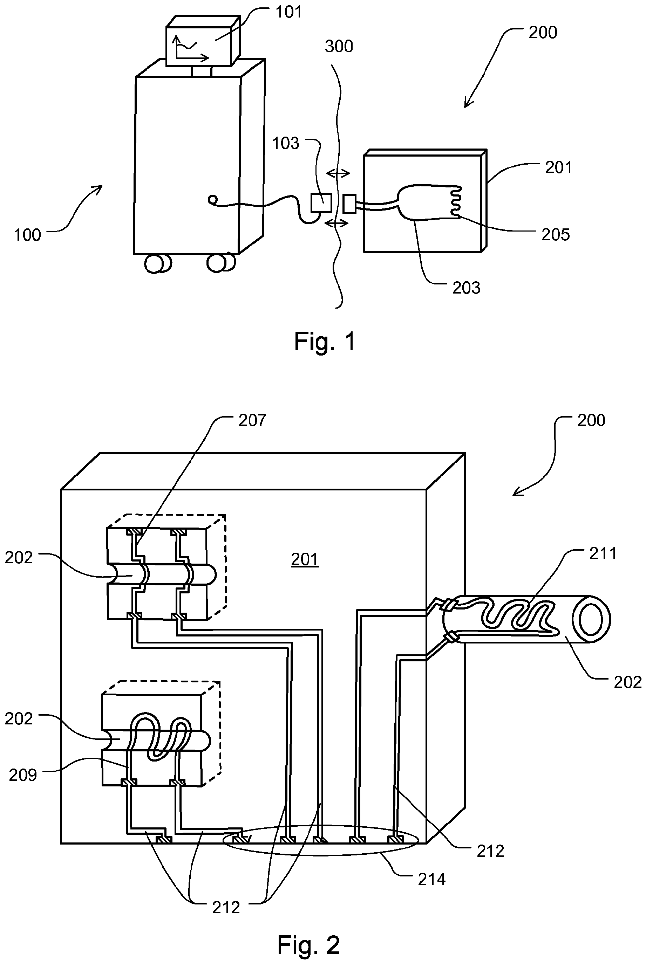

**Fig. 1** zeigt sehr schematisch eine erfindungsgemäße Behandlungsvorrichtung, die nur beispielhaft als Blutbehandlungsvorrichtung 100 ausgeführt ist, mit einer erfindungsgemäßen medizinischen Einrichtung 200 (kurz: Einrichtung 200).

Die Einrichtung 200 ist exemplarisch als ein Disposable ausgestaltet. Sie wurde mittels eines additiven, maskierungsfreien Druckverfahrens teilfunktionalisiert.

Die Blutbehandlungsvorrichtung 100 und die erfindungsgemäße Einrichtung 200 sind über eine Schnittstelle 300 miteinander in Signalkommunikation verbunden.

Die Einrichtung 200 weist ein Hartteil 201 auf. Auf dem Hartteil 201 sind Teile einer Sensoranordnung vorgesehen, hier eine Leiterbahn 203 und ein Wandler 205.

Der Wandler 205 kann beispielsweise ein Druckaufnehmer sein. Er kann auf das Hartteil 201 aufgedruckt sein. Er kann disposableseitig lediglich die zu messende Größe "Druck" in ein analoges elektrisches Signal wandeln.

Das elektrische Signal wird über die ebenfalls additiv aufgedruckte Leiterbahn 203 zu der definierten Schnittstelle 300 geleitet, welche in Verbindung mit einer maschinenseitigen Auswerteeinheit, angedeutet durch einen Monitor 101 zur Darstellung der mittels der Auswerteeinheit erzielten Auswerteergebnisse, steht.

Maschinenseitig kann das Signal mittels eines AD (Analog-Digital-)-Wandlers oder AD-Converters 103 digitalisiert werden, es können Schritte der Nachverarbeitung (Filter, Glättung, Fouriertransformation, Zero-Filling etc.) erfolgen, bevor schließlich eine Auswertung und Interpretation erfolgt. Alle diese optionalen Schritte können z. B. in der Auswerteeinheit durchgeführt werden.

**Fig. 2** zeigt eine medizinische Einrichtung 200 gemäß einer zweiten beispielhaften Ausführungsform der vorliegenden Erfindung.

Die medizinische Einrichtung 200 ist hier wiederum ein teilfunktionalisiertes Disposable, mit drei unterschiedlichen Wandlern 207, 209 und 211.

Oben links ist in Fig. 2 beispielhaft ein Wandler 207 zur Messung der Leitfähigkeit angeordnet. In der vorliegenden, vereinfachten Darstellung besteht dieser aus zwei Leiterbahnen, welche erfindungsgemäß mittels des additiven, maskierungsfreien Druckverfahrens im Inneren eines fluidführenden Kanals 202 der Einrichtung 200 aufgebracht sind.

Die beiden anderen Wandler 209, 211 zeigen beispielhafte Ausführungen von Druckaufnehmern. Diese können als Dehnungsmessstreifen an der Innenkontur (siehe den Wandler 209) oder an der Außenkontur (siehe den Wandler 211) des fluidführenden Kanals oder Schlauchs 202 der Einrichtung 200 angebracht sein.

Über die Wandler 209, 211 hinaus zeigt Fig. 2 beispielhaft die elektromagnetische Signalführung mit Leiterbahnen 212 zur Maschinenschnittstelle 300, hier beispielhaft einen Multipolstecker 214. Die Leiterbahnen 212 zur Signalleitung können hierbei mit demselben additiven und maskierungsfreien Verfahren, insbesondere im selben Verfahrensschritt, auf die planare Fläche des Hartteils 201 aufgebracht sein.

Es sind aber auch nicht-planare (dreidimensionale) Leitungsführungen im oben beschriebenen Sinn oder Kreuzungen von (entsprechend isolierten) Leitungen möglich.

Die Leiterbahnen 212 können auch mittels einer zweiten, ebenfalls additiven und maskierungsfreien Drucktechnik aufgebracht werden, z. B. in einem zweiten Produktionsschritt, der dem Aufbringen der Wandler 207, 209, 211 folgt. Die Gesamtheit der Leiterbahnen 212 zur Signalführung vom Ort des jeweiligen Wandlers 207, 209, 211, welcher die zu messende Größe z. B. in ein elektro-magnetisches Signal wandelt, das mittels der Leiterbahnen 212 zur Maschinenschnittstelle 300 geführt wird, kann auch einzelne Teile beinhalten, die mittels Zweikomponenten-Spritzguss von leitfähigen Polymeren aufgebracht sind.

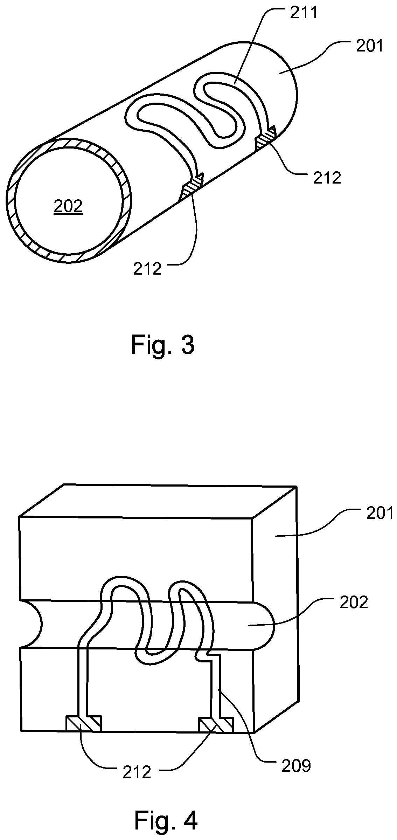

**Fig. 3** und **Fig. 4** zeigen die zu Fig. 2 diskutierten Druckwandler 209 und 211 in einer vergrößerten Perspektive. Sie sind nur exemplarisch als Dehnungsmessstreifen ausgestaltet. Es ist zu erkennen, dass die Wandler 207, 209, 211 mit dem erfindungsgemäßen additiven und maskierungsfreien Druckverfahren nicht nur in einer planaren Fläche aufgebracht werden, sondern dass diese auch in der dritten Dimension über beliebige topologische Begebenheiten geführt werden können.

So laufen die Strukturen des innen additiv aufgebrachten Druckwandlers 209 über die Kante der Oberfläche der dargestellten Hälfte der Einrichtung 200 und dann entlang der konkaven Aushöhlung des Fluidkanals 202 sowie auf der gegenüberliegenden Seite erneut über die Kante an der Oberfläche.

Der außen aufgebrachte Druckwandler 211 folgt der zylindrischen Oberflächentopologie des fluidführenden Kanals oder Schlauchs 202. Diese Möglichkeit, nicht nur planare Oberflächen, sondern auch beliebige dreidimensionale Topologien mit Elektroden, Leiterbahnen usw. zu beschichten, stellt einen weiteren Vorteil für die im erfindungsgemäßen, additiven und maskierungsfreien Druckverfahren verwendeten Techniken dar.

Der Multipolstecker 214, welcher an der Schnittstelle 300 zur Maschinenseite für alle von den einrichtungsseitig angebrachten Wandlern 207, 209, 211 übermittelten Signalen dienen kann, kann ebenfalls mit einer erfindungsgemäßen additiven, maskierungsfreien Drucktechnik aufgebracht sein.

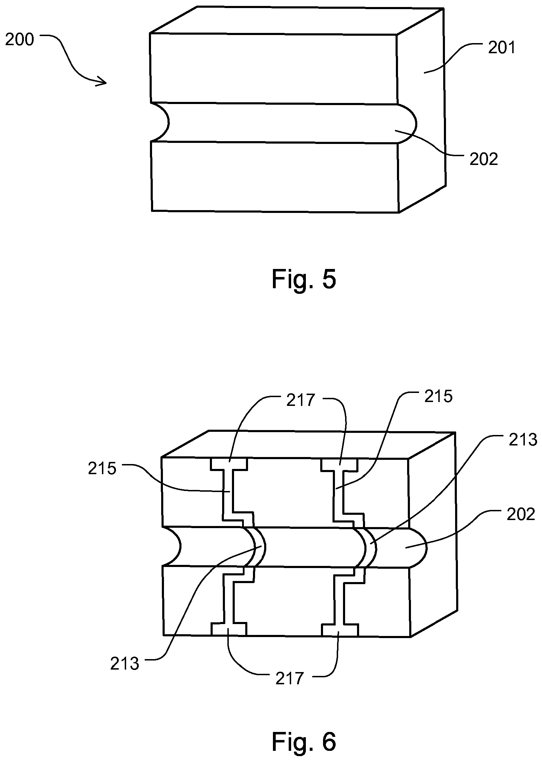

**Fig. 5** zeigt ein Ausgangsprodukt des erfindungsgemäßen Fertigungsprozesses zum Erstellen eines teilfunktionalisierten Disposables gemäß der vorliegenden Erfindung.

Es kann sich hierbei um ein Hartteil 201 handeln, das mittels Spritzguss erzeugt wurde. Fig. 5 zeigt ferner eine anteilig vom Hartteil 201 gebildete Fluidbahn 202.

Das Hartteil 201 weist noch keinen Wandler auf. Dieser wird in einem sich anschließenden Aufbringungsverfahren aufgebracht. Das Ergebnis der Aufbringung ist in Fig. 6 zu sehen.

**Fig. 6** zeigt das Hartteil 201 der Fig. 5 nach additivem Aufbringen eines Wandlers 213 mit zwei Leiterbahnen 215 sowie Kontakten 217, z. T. durch die gewölbte Kontur der Fluidbahn 202 hindurch.

Weitere Schritte des erfindungsgemäßen Produktionsverfahrens - hier nicht gezeigt - können weitere Nachbearbeitungsschritte an den Elektroden enthalten, wie z. B. Schleifen, Polieren, Isolieren, Aufbringen weiterer funktionaler Schichten aus anderem Material usw.

Schließlich kann die Bedruckung der Signalanbindung an die Maschinenschnittstelle sowie die Bedruckung des Multipolsteckers selbst erfolgen. Sowohl die Leiterbahnen als auch der Multipolstecker können planar oder dreidimensional mit einer oder mehreren der erfindungsgemäßen additiven und maskierungsfreien Drucktechniken aufgebracht werden. Auch hier sind ein oder mehrere Nachbearbeitungsschritte, wie zuvor bei den Elektroden beschrieben, denkbar.

Ferner können zwei oder mehr derart präparierte Disposableteile, wobei auch nur einzelne davon die Komponenten der erfindungsgemäßen (Teil-)Funktionalisierung enthalten können, zusammengefügt werden. Kombiniert können etwa zwei Disposablehälften im Bereich der Fluidkanäle einen geschlossenen Kanal ergeben, mit einem kreisförmigen Querschnitt. Weiterhin sind auch andere Kanalgeometrien und Querschnittsformen denkbar. Die zweite Disposablehälfte kann auch nur ein flacher Deckel sein, sodass alle notwendigen Vertiefungen auf der ersten Hälfte angeordnet sind.

Die zweite Hälfte kann auch aus einer Membran bestehen. Anstatt einer zweiten Disposablehälfte kann auch eine Versiegelung oder Isolierung der Oberfläche der ersten Hälfte mittels einer oder mehrerer zusätzlicher Schichten aufgebracht sein. Diese Schichten können gedruckt sein. Diese Schichten können auch mit einem anderen bekannten Verfahren produziert und dann aufgebracht sein. Alternativ kann die Isolierung bzw. die Versiegelung auch in einem weiteren Spritzgussschritt durch Umspritzen des bedruckten Hartteils aufgebracht werden.

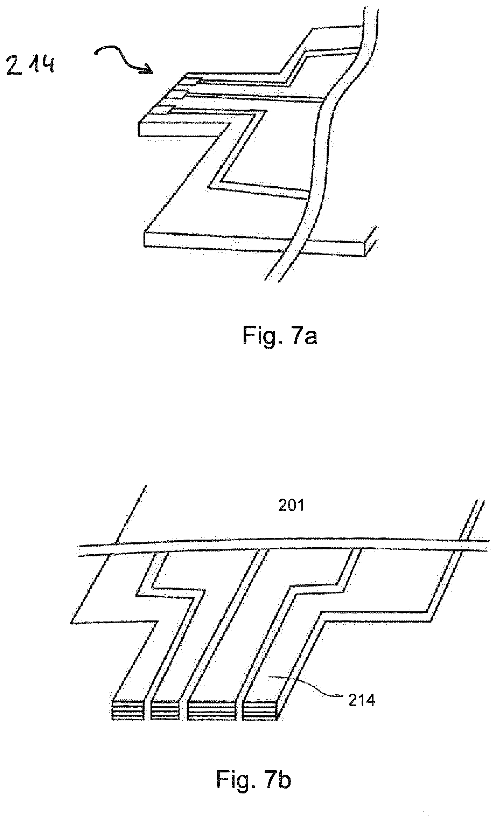

**Fig. 7a** zeigt wie **Fig. 7b** den o. g. Multipolstecker 214 oder Multipolverbinder, welcher zu Fig. 2 bereits diskutiert wurde, in verschiedenen Ansichten.

### Bezugszeichenliste

- 100: Blutbehandlungsvorrichtung
- 101: Monitor
- 103: AD-Wandler

- 200: Blutkassette als Beispiel einer medizinischen Einrichtung
- 201: Kassettenkörper oder Kassettengrundkörper, Hartteil; Hartkörper, Substrat
- 202: Fluidbahn, Kanal, Strömungskanal
- 203: Leiterbahn oder Signalleiter
- 205: Wandler
- 207: Wandler
- 209: Wandler
- 211: DMS-Element als Wandler
- 212: Leiterbahn oder Signalleiter
- 213: Wandler
- 214: Multipolstecker, Multipolverbinder
- 215: Leiterbahn
- 217: Kontakt

- 300: Schnittstelle, Maschinenschnittstelle

## Patentansprüche

1. Medizinische Einrichtung (200) mit jeweils wenigstens
- einem Hartteil (201) mit Fluidbahnen (202) zum Führen eines medizinischen Fluids, insbesondere Blut, durch das Hartteil (201);
- einem Wandler (205, 207, 209, 211, 213), wobei der Wandler (205, 207, 209, 211, 213) angeordnet ist zum Messen einer Eigenschaft des medizinischen Fluids, während dieses in einer der Fluidbahnen (202) vorliegt;
wobei der Wandler (205, 207, 209, 211, 213) zumindest in einem Abschnitt hiervon mittels wenigstens eines additiven Aufbringungsverfahrens, vorzugsweise eines Druckverfahrens, auf das Hartteil (201) aufgebracht ist, **dadurch gekennzeichnet, dass** der Wandler (205, 207, 209, 211, 213) zum Messen oder Bestimmen von Druck konfiguriert oder ausgestaltet ist.

2. Medizinische Einrichtung (200) nach Anspruch 1, wobei das Aufbringungsverfahren das Aufbringen von leitfähiger Tinte umfasst.

3. Medizinische Einrichtung (200) nach einem der vorangegangenen Ansprüche, wobei die medizinische Einrichtung (200) eine Vielzahl von Wandlern (205, 207, 209, 211, 213) aufweist, welche jeweils zumindest in einem Abschnitt hiervon mittels additiven Aufbringungsverfahrens, vorzugsweise eines Druckverfahrens, auf das Hartteil (201) aufgebracht sind.

4. Medizinische Einrichtung (200) nach einem der vorangegangenen Ansprüche, wobei mittels additiven Aufbringungsverfahrens weitere Abschnitte einer den Wandler (205, 207, 209, 211, 213) umfassenden Sensoranordnung oder weiterer Sensoranordnungen mittels Einsatzes desselben Aufbringungsverfahrens aufgebracht wurden.

5. Medizinische Einrichtung (200) nach Anspruch 3, wobei die mittels additiven Aufbringungsverfahrens aufgebrachten Wandler (205, 207, 209, 211, 213) und/oder weiteren Abschnitte der zugehörigen Sensoranordnungen im selben Produktionsschritt aufgebracht wurden.

6. Medizinische Einrichtung (200) nach einem der vorangegangenen Ansprüche, wobei die mittels additiven Aufbringungsverfahrens aufgebrachten Abschnitte zusätzlich zu dem wenigstens einen Wandler (205, 207, 209, 211, 213) jeweils wenigstens Leiterbahnen (212, 215) oder einen Multipolstecker (214) oder eine Vielzahl hiervon umfassen.

7. Medizinische Einrichtung (200) nach einem der vorangegangenen Ansprüche, weiter aufweisend einen oder mehrere Wandler (205, 207, 209, 211, 213), welche zum Messen oder Bestimmen von Leitfähigkeit, Spannung oder Strom konfiguriert oder ausgestaltet sind.

8. Medizinische Einrichtung (200) nach einem der vorangegangenen Ansprüche, wobei ein Multipolstecker (214) ebenfalls mit erstem Aufbringungsverfahren aufgebracht wurde.

9. Medizinische Einrichtung (200) nach einem der vorangegangenen Ansprüche, wobei zum Aufbringen der mittels additiver Aufbringungsverfahren aufgebrachten Abschnitte wenigstens ein zweites Aufbringungsverfahren zum Einsatz gekommen ist.

10. Medizinische Einrichtung (200) nach einem der vorangegangenen Ansprüche, wobei Abschnitte des oder der Wandler (205, 207, 209, 211, 213) und/oder der weiteren Abschnitte mittels Spritzgussverfahren aufgebracht wurden, z. B. Zwei-Komponentenspritzguß, wobei eine Komponente leitfähige Polymere umfasst.

11. Medizinische Einrichtung (200) nach einem der vorangegangenen Ansprüche, wobei die medizinische Einrichtung (200) eine Blutkassette ist.

12. Verfahren zum Herstellen einer medizinischen Einrichtung (200) nach einem der vorangegangenen Ansprüche, mit den Schritten:
- Bereitstellen eines Hartteils (201) der medizinischen Einrichtung (200) mit einem Fluidsystem für ein medizinisches Fluid;
**gekennzeichnet, durch den Schritt**
- Aufbringen wenigstens eines Wandlers (205, 207, 209, 211, 213) zumindest in einem Abschnitt hiervon mittels additiven Aufbringungsverfahrens, vorzugsweise eines Druckverfahrens, auf das Hartteil (201), wobei der Wandler (205, 207, 209, 211, 213) zum Messen oder Bestimmen von Druck konfiguriert oder ausgestaltet ist.

13. Verfahren nach Anspruch 12, weiter umfassend wenigstens einen der folgenden Schritte:
- Nachbearbeitungsschritte, insbesondere an Leiterbahnen (212, 215), wie z. B. Schleifen, Polieren, Isolieren, Aufbringen weiterer funktionaler Schichten aus anderem Material;
- additives Aufbringen, z. B. durch Aufdrucken, der Signalanbindung an die Maschinenschnittstelle (300);
- additives Aufbringen, z. B. durch Aufdrucken, des Multipolsteckers (214); und
- Zusammenfügen von zwei oder mehr mittels additiven Aufbringens bearbeiteter Abschnitte der medizinischen Einrichtung (200).

14. Behandlungsvorrichtung (100), verbunden mit einer medizinischen Einrichtung (200) gemäß einem der Ansprüche 1 bis 11, wobei ein Multipolverbinder der Behandlungsvorrichtung (100) mit einem Multipolstecker (214) der medizinischen Einrichtung (200) verbunden ist.

15. Behandlungsvorrichtung (100) nach Anspruch 14, ausgestaltet als Blutbehandlungsvorrichtung, insbesondere als Apheresevorrichtung oder Dialysevorrichtung, insbesondere als Hämofiltrationsvorrichtung, als Hämodiafiltrationsvorrichtung, als Filtrationsvorrichtung oder als Vorrichtung zum extrakorporalen Gasaustausch.

## Claims

1. A medical device (200) comprising at least, respectively:
- a hard part (201) with fluid paths (202) for guiding a medical fluid, in particular blood, through the hard part (201);
- a transducer (205, 207, 209, 210, 211, 213), the transducer (205, 207, 209, 210, 211, 213) being arranged to measure a property of the medical fluid when it is present in one of the fluid paths (202),
wherein the transducer (205, 207, 209, 210, 211, 213) is, at least in one section thereof, applied to the hard part (201) by means of at least one additive application method, preferably by means of a printing method,
**characterized in that** the transducer (205, 207, 209, 210, 211, 213) is configured or designed to measure or to determine a pressure.

2. The medical device (200) according to claim 1, wherein the application method includes the application of conductive ink.

3. The medical device (200) according to anyone of the preceding claims, wherein the medical device (200) comprises a plurality of transducers (205, 207, 209, 210, 211, 213), each of which being respectively, at least in one section thereof, applied to the hard part (201) by means of an additive application method, preferably by means of a printing method.

4. The medical device (200) according to anyone of the preceding claims, wherein, by means of an additive application method, further sections of a sensor arrangement comprising the transducer (205, 207, 209, 211, 213) or additional sensor arrangements were applied by using the same application method.

5. The medical device (200) according to claim 3, wherein the transducers (205, 207, 209, 211, 213) which were applied by means of the additive application method and/or further sections of the associated sensor arrangements were applied in the same production step.

6. The medical device (200) according to anyone of the preceding claims, wherein the sections applied by means of the additive application method, as well as additionally to the at least one transducer (205, 207, 209, 211, 213) each comprise at least conductor paths (212, 215) or a multi-pole plug (214) or a plurality thereof.

7. The medical device (200) according to anyone of the preceding claims, wherein the transducer or the transducers (205, 207, 209, 210, 211, 213) is/are configured or designed to measure or determine conductivity, voltage or current.

8. The medical device (200) according to anyone of the preceding claims, wherein a multi-pole plug (214) was also applied using the first application method.

9. The medical device (200) according to anyone of the preceding claims, wherein at least a second application method has been used to apply the sections applied by means of the additive application methods.

10. The medical device (200) according to anyone of the preceding claims, wherein sections of the transducer or of the transducers (205, 207, 209, 211, 213) and/or further sections have been applied by means of an injection molding method, for example two-components injection molding, wherein one component comprises conductive polymers.

11. The medical device (200) according to anyone of the preceding claims, wherein the medical device (200) is a blood cassette.

12. A method for producing a medical device (200) according to anyone of the preceding claims, comprising the following steps:
- providing a hard part (201) of the medical device (200) with a fluid system for a medical fluid;
**characterized by** the following step:
- applying at least one transducer (205, 207, 209, 211, 213) to the hard part (201) by means of an additive application method, preferably by means of a printing method, in at least one section thereof, wherein the transducer (205, 207, 209, 211, 213) is configured or designed to measure or determine a pressure.

13. The method according to claim 12, further comprising at least one of the following steps:
- post-processing steps, in particular on conductor paths (212, 215), such as for example grinding, polishing, isolating, applying further functional layers made of other material;
- additive application, for example by printing, of the signal connection to the machine interface (300);
- additive application, for example by printing, of the multi-pole plug (214); and
- assembly of two or more sections of the medical device (200) processed by means of additive application.

14. A treatment apparatus (100) connected to a medical device (200) according to anyone of claims 1 to 11, wherein a multi-pole connector of the treatment apparatus is connected to a multi-pole plug (214) of the medical device.

15. The treatment apparatus (100) according to claim 14, designed as a blood treatment apparatus, in particular as an apheresis device or a dialysis device, in particular as a hemofiltration device, a hemodiafiltration device, a filtration device or a device for extracorporeal gas exchange.

## Revendications

1. Un dispositif médical (200) comprenant respectivement au moins:
- une partie rigide (201) avec des passages pour fluide (202) prévus pour guider un fluide médical, notamment du sang, au travers de la partie rigide (201);
- un transducteur (205, 207, 209, 210, 211, 213), le transducteur (205, 207, 209, 210, 211, 213) étant agencé de façon à mesurer une propriété du fluide médical quand celui-ci est présent dans l'un des passages pour fluide (202);
où le transducteur (205, 207, 209, 210, 211, 213) est, au moins dans une section de celui-ci, appliqué sur la partie rigide (201) au moyen d'un procédé d'application additive, de préférence au moyen d'un procédé d'impression,
**caractérisé en ce que** le transducteur (205, 207, 209, 210, 211, 213) est configuré ou conçu pour mesurer ou déterminer une pression.

2. Le dispositif médical (200) selon la première revendication, où le procédé d'application comprend l'application d'une encre conductrice.

3. Le dispositif médical (200) selon l'une quelconque des revendications précédentes, où le dispositif médical (200) comprend une pluralité de transducteurs (205, 207, 209, 210, 211, 213), où chacun, au moins dans une section de celui-ci, est appliqué sur la partie rigide (201) au moyen d'un procédé d'application additive, de préférence au moyen d'un procédé d'impression.

4. Le dispositif médical (200) selon l'une quelconque des revendications précédentes, où, au moyen d'un procédé d'application additive, des sections supplémentaires d'un agencement de capteurs comprenant le transducteur (205, 207, 209, 211, 213) ou d'autres agencements de capteurs ont été appliquées/és en utilisant le même procédé d'application additive.

5. Le dispositif médical (200) selon la revendication 3, où les transducteurs (205, 207, 209, 211, 213) appliqués au moyen du procédé d'application additive, et/ou d'autres sections des agencements de capteurs associés, ont été appliqués dans la même étape de production.

6. Le dispositif médical (200) selon l'une quelconque des revendications précédentes, où les sections appliquées au moyen de la méthode d'application additive, en plus d'au moins un transducteur (205, 207, 209, 211, 213), comprennent, chacune, au moins des pistes conductrices (212, 215) ou une fiche multi-pôles (214) ou une pluralité de ceux-ci.

7. Le dispositif médical (200) selon l'une quelconque des revendications précédentes, où le ou les transducteur/s (205, 207, 209, 211, 213) est/sont configuré/s ou conçu/s pour mesurer ou déterminer la conductivité, la tension ou le courant.

8. Le dispositif médical (200) selon l'une quelconque des revendications précédentes, où une fiche multi-pôles (214) a également été appliquée en utilisant le premier procédé d'application.

9. Le dispositif médical (200) selon l'une quelconque des revendications précédentes, où au moins un second procédé d'application a été utilisé pour appliquer les sections appliquées au moyen de procédés d'application additive.

10. Le dispositif médical (200) selon l'une quelconque des revendications précédentes, où des sections du ou des transducteurs (205, 207, 209, 211, 213) et/ou des sections supplémentaires ont été appliquées au moyen d'un procédé de moulage par injection, par exemple un moulage par injection à deux composants, un composant comprenant des polymères conducteurs.

11. Le dispositif médical (200) selon l'une quelconque des revendications précédentes, où le dispositif médical (200) est une cassette à sang.

12. Un procédé pour fabriquer un dispositif médical (200) selon l'une quelconque des revendications précédentes, comprenant les étapes suivantes:
- la fourniture d'une partie rigide (201) du dispositif médical (200) avec un système fluidique pour un fluide médical;
**caractérisé par** l'étape suivante:
- l'application d'au moins un transducteur (205, 207, 209, 211, 213), au moins dans une section de celui-ci, sur la partie rigide (201) au moyen d'un procédé d'application additive, de préférence au moyen d'un procédé d'impression, où le transducteur (205, 207, 209, 211, 213) est configuré ou conçu pour mesurer ou déterminer une pression.

13. Le procédé selon la revendication 12, comprenant en outre au moins une des étapes suivantes:
- des étapes de post-traitement, en particulier sur les pistes conductrices (212, 215), comme par exemple le meulage, le polissage, l'isolation, l'application de couches fonctionnelles supplémentaires d'un autre matériau;
- l'application additive, par exemple par impression, de la connexion du signal à l'interface de la machine (300);
- l'application additive, par exemple par impression, de la fiche multi-pôles (214); et
- l'assemblage de deux ou de plusieurs sections du dispositif médical (200) traitées au moyen d'une application additive.

14. Un appareil de traitement (100) relié à un dispositif médical (200) selon l'une quelconque des revendications 1 à 11, où un connecteur multi-pôles de l'appareil de traitement est relié à une fiche multi-pôles (214) du dispositif médical.

15. L'appareil de traitement (100) selon la revendication 14, conçu comme un appareil de traitement du sang, notamment comme appareil d'aphérèse ou appareil de dialyse, notamment comme appareil d'hémofiltration, appareil d'hémodiafiltration, appareil de filtration ou appareil d'échange gazeux extracorporel.
